(19) Europäisches Patentamt
European Patent Office
Office européen des brevets

(11) Publication number: **0 408 301 A1**

# (12) EUROPEAN PATENT APPLICATION

(21) Application number: **90307513.3**

(22) Date of filing: **10.07.90**

(51) Int. Cl.5: **C12N 15/62**, C12N 15/86, C12N 15/39

(30) Priority: **11.07.89 GB 8915870**

(43) Date of publication of application: **16.01.91 Bulletin 91/03**

(84) Designated Contracting States: **GR**

(71) Applicant: **NATIONAL RESEARCH DEVELOPMENT CORPORATION**
**101 Newington Causeway**
**London SE1 6BU(GB)**

(72) Inventor: **Gaffney, Dairena Frances**
**93 Norse Road**
**Glasgow G14 9EF(GN)**
Inventor: **Patel, Arvind Hirabhai**
**5 Aikman Road, Motherwell**
**Lanarkshire ML1 3BT, Scotland(GB)**
Inventor: **Stow, Nigel Dennis**
**48 Lamington Road**
**Glasgow G52 2SE, Scotland(GB)**
Inventor: **Subak-Sharpe, John Herbert**
**17 Kingsborough Gardens**
**Glasgow G12 9NH, Scotland(GB)**

(74) Representative: **Percy, Richard Keith**
**Patent Department National Research Development Corporation 101 Newington Causeway**
**London SE1 6BU(GB)**

## (54) DNA coding for a polypeptide signal sequence in vaccinia virus.

(57) Signal sequence DNA encodes the following amino acid sequence (SEQUENCE ID. NO. 1):

```
Met Lys Gln Tyr Ile Val Leu Ala Cys Met
Cys Leu Ala Ala Ala Ala Met Pro Ala Ser
```

or a conservatively modified variant of said amino acid sequence. It is useful for incorporation into recombinant DNA constructs of a poxvirus and a foreign gene in order to facilitate secretion of the expression product of the foreign gene.

EP 0 408 301 A1

# DNA CODING FOR A POLYPEPTIDE SIGNAL SEQUENCE IN VACCINIA VIRUS

## Background of the invention

### 1. Field of the invention

This invention is in the recombinant DNA field and relates to DNA coding for the secretion signal sequence of a polypeptide expressed by a strain of vaccinia virus.

### 2. Description of the prior art

In recent years vaccinia virus has been used as a vector for introducing a foreign gene into mammalian cells. The foreign gene is introduced into the virus in a "non-essential region" within its genome in which it does not interfere with essential functions of virus, so that the virus is capable of at least limited, if not full, replication in the mammalian host. Transcription of the foreign gene requires a vaccinia virus promoter, which could be obtained by excising the relevant DNA from elsewhere in the genome, to be co-inserted upstream of the foreign gene within the non-essential region.

The vaccinia virus (VV) promoter most widely used is the "7.5K" promoter (P7.5K). It promotes the transcription of a gene (7.5K gene) which encodes a protein of relative molecular mass of about 7.5 kiloDaltons. The gene and its promoter have been partially sequenced by S. Venkatesan et al ., Cell 25 , 805-813 (1981).

There is considerable interest in improving the expression of VV recombinants.

The major research on VV which has led, for example, to its use as the vector for a rabies virus vaccine, has been performed almost exclusively with the Western Reserve (WR) strain.

Over ten years ago, working with the Evans strain, M.A. McCrae and T.H. Pennington, Journal of Virology 28 , 828-834 (1978), described a VV polypeptide of r.m.m. approximately 35 kD which was secreted into the cell culture medium in large amounts at 2 to 2.5 hours post-infection and continued to be secreted. However, one of the present inventors found that the Evans strain did not grow well. The polypeptide has never been identified in the commonly used WR strain. [Working with the WR strain, G.J. Kotwal and B. Moss, Nature 335 , 176-178 (1988) have reported the purification of a 35kD polypeptide and DNA sequencing of a gene coding for it, but they have actually described a different 35kD polypeptide to that reported by McCrae and Pennington.].

## Summary of the invention

The inventors' research was founded on the discovery that the 35kD polypeptide is secreted from the Lister strain, which does grow well and on the hope that the Lister strain of VV might contain a powerful promoter for the 35kD polypeptide. Such a promoter might be useful for linking to a heterologous gene to enhance expression of that gene in VV recombinants, i.e. to improve on the 7.5K promoter.

Upon cloning and sequencing the "35K" gene, however, it was found that the promoter sequence was very nearly identical with that of the 7.5K promoter of WR strain.

However, it was found that the 35K gene in the Lister strain contains a signal sequence. A signal sequence is one which enables the polypeptide to pass through the cell membrane of the infected cell and enter the culture medium. During secretion, the signal sequence is cleaved, resulting in production of mature protein in the culture medium. In the present instance a putative signal sequence was discovered initially by sequencing the 35K gene and comparing its sequence with that of the 7.5K gene.

The first twelve codons of the open reading frame for the gene are the same for the two viruses, but thereafter they diverge. The putative signal sequence of the 35K gene comprises the first 17 codons of the open reading frame, for the 35K gene. The first 20 codons of this ORF are shown below:

ATG AAA CAA TAT ATC GTC CTG GCA TGC ATG
Met Lys Gln Tyr Ile Val Leu Ala Cys Met
TGC CTG GCG GCA GCT GCT ATG CCT GCC AGT
Cys Leu Ala Ala Ala Ala Met Pro Ala Ser

(See also SEQUENCE ID NO: 1 which differs only in that the underlining is omitted and numbering is added). The underlined portion represents bases which are not present in the corresponding DNA of the WR strain. The 35K gene (a term used herein to denote the gene from which the 35kD protein is translated) codes differently from the 7.5K gene, not only in that it contains these additional bases, but also in that it is frameshifted immediately downstream of the additional sequence. The references herein to a relative molecular mass of 35kD for the mature protein are labels of convenience and not to be construed as denoting that this mass is necessarily accurate. A precise r.m.m. is calculable from the amino acid sequence shown hereinafter.

The present invention provides a DNA molecule which is suitable for use in the manufacture of a cassette, ultimately for insertion into the genome of a poxvirus, said DNA molecule encoding the following amino acid sequence (SEQUENCE ID NO: 2):

| | | | | | | | | | |
|---|---|---|---|---|---|---|---|---|---|
| Met | Lys | Gln | Tyr | Ile | Val | Leu | Ala | Cys | Met |
| Cys | Leu | Ala | Ala | Ala | Ala | Met | Pro | Ala | Ser |

This sequence which might further contain one, two, three of four additional amino acids (following on from the C-terminal end of SEQUENCE ID NO: 2), i.e. Leu, possibly Gln, possibly another Gln and possibly thereafter Ser, is herein referred to as "the signal sequence DNA". The term "signal sequence" is used notwithstanding that it is probable that only the first 17 codons are required in secretion of she 35kD protein, at least 3 additional codons encoding amino acids of the mature 35kD protein being considered essential for the use of the signal sequence in a recombinant vector.

The signal sequence DNA can be varied by appropriate substitutions of amino acids, particularly in the hydrophobic region from Ile onwards by hydrophobic substitutes (Gly, Ala, Val, Leu, Ile, Cys, Met, Phe, Tyr, Trp, Pro, His and sometimes Lys). The permissible extent of such substitution can be determined by experiment.

The cassette will preferably include a promoter, desirably a strong promoter, for example the 7.5K promoter or the promoter which precedes the 35K gene. It will preferably also include a multiple cloning site downstream of the signal sequence, so that a foreign gene can easily be inserted.

The foreign gene to be introduced into the mammalian cells can be in principle any which is foreign with respect to the poxvirus with which the cells are to be infected. The cassette conveniently also contains, therefore, the foreign gene in frame with the signal sequence DNA.

A preferred cassette therefore comprises the following elements linked together so that transcription can occur:-[promoter (such as VV 7.5K)]-["signal sequence DNA"]-[optionally further downstream sequence from the 35K gene, e.g. DNA encoding SEQUENCE ID NO: 3, but bearing in mind that DNA encoding the first 4 amino acids of SEQUENCE ID NO: 3 could be part of the signal sequence DNA and would not then be repeated here]-[foreign gene or a multiple cloning site for insertion of the foreign gene]-[a viral transcription termination signal].

The invention includes a vector, such as a bacterial or yeast plasmid, carrying the cassette DNA together with flanking poxvirus sequence at each end thereof (from the infecting poxvirus). This is referred to herein as a "recombination vector", meaning a vector which is ultimately destined for use in the homologous recombination process. In the homologous recombination, the flanking poxvirus sequence in the recombination vector interchanges with the corresponding sequence in the "parent" strain of the infecting poxvirus, whereby the cassette DNA becomes inserted into that poxvirus.

Ideally, three separate plasmids of this type are provided with multiple cloning sites for insertion of the foreign gene into whichever of the three possible reading frames will put it in frame with the signal sequence.

Further, the invention includes a recombinant poxvirus resulting from homologous recombination and therefore containing the cassette DNA, animal cells infected with the recombinant poxvirus, the foreign protein obtained directly or indirectly from recombinant infected cells by in vitro culture, and, where national patent law permits, a method of vaccination which comprises administering the recombinant virus to an animal subject.

Brief description of the drawings

Figure 1 is a restriction map of part of the vaccinia virus (Lister strain) genome, showing the location of the 35K gene;

Figure 2 is a plasmid diagram showing schematically the construction of a preferred cassette for use in preparing a recombination vector of the invention;

Figure 3 (= 3a + 3b) is a linearised plasmid derivation chart showing the construction of recombination vectors of the invention containing cassette DNA including the signal sequence, along with another recombination vector made for comparative purposes (and which are ready for homologous recombination into the 35K or 7.5K gene of vaccinia virus); and

Figure 4 (= 4a + 4b) is another linearised plasmid derivation chart showing the construction of recombination vectors of the invention and comparative recombination vectors ready for homologous recombination intc the TK gene of vaccinia virus.

Description of the preferred embodiments

The invention is of interest in relation to poxvirus vectors generically. The poxvirus family have sufficient similarity to allow the VV 7.5K promoter to be used in fowlpox, capripox cowpox, and dovepox. Although the invention is hereinafter described with reference to VV, it will be appreciated that the promoter and signal sequence combination is likely to be useful in aiding gene expression and prcduct secretion in any other poxvirus in which the 7.5K promoter is functional for transcription of its genomic DNA.

It is intended primarily that the signal sequence of the invention will be used in association with the 35X promoter DNA or its 7.5K counterpart which precedes it. It is known from Cochran et al ., J. Virol 54 , 30-37, (1985) that the 137 bp of VV DNA immediately upstream from the ATG start codon includes both the early and late promotion functions of the 7.5K promoter in WR strain. Of these 137 bp, the last 18 (counting backwards) were not present in the constructs described by Cochran et al ., and are therefore presumably non-essential. The 35K promoter in the Lister strain is almost identical, having only two base substitutions within the 86 bp length upstream of the putative mRNA start site. It is highly likely that the 137-18 115 bp length could be shortened, if desired, and various deletions or changes made in the promoter sequence, as may be determined by experiment, e.g. deletion mapping, synthesis of oligonucleotides, site-directed mutagenesis etc. as well known in the art. All such variant sequences are within the scope of the term "7.5K promoter" (P7.5K) or "35K promoter" (P35K) as used herein.

It is possible, however, that the signal sequence could be used in poxviruses in conjunction with other poxvirus promoters or in other viruses together with their respective promoters, e.g. in herpes simplex virus.

The promoter need not be a strong one, as the signal sequence will ensure secretion irrespective of promoter strength.

That part of the precursor polypeptide of the 35kD protein shown above which is cleaved after secretion ends with a methionine (Met) residue, the proline (Pro) residue which immediately follows being the N-terminal amino acid of the mature 35kD protein. In this invention, any 17-codon DNA coding for the signal sequence of the precursor polypeptide is coupled with DNA coding for at least the three N-terminal amino acids, preferably at least the five N-terminal amino acids, of the mature 35kD protein, and followed by the desired foreign gene (exogeneous with respect to the poxvirus). This will normally ensure that the foreign protein is secreted by the host cells. Thus, the protein secreted will be the foreign protein fused at its N-terminus to amino acids from the N-terminal portion of the mature 35kD protein. The desirable number of amino acids from the N-terminus of the mature 35kD protein is a matter for experiment, but expression of a fusion polypeptide has been obtained when using a site in the 22nd or 42nd codon of the reading frame (including the signal sequence) for fusion of the foreign gene.

It is possible to insert the signal sequence after any poxvirus promoter, the 35K (or 7.5K) promoter not being critical. It is not even critical that the promoter be from the same virus. The invention includes any method of insertion, e.g. by creating a cassette containing the promoter and signal sequence DNA or by introducing the signal sequence DNA separately. Indeed, site-directed mutagenesis could be used to alter existing DNA.

Currently, it is considered best to make a construct comprising a non-essential region of the poxvirus genome interrupted by or containing the cassette DNA, i.e. the promoter, the signal sequence, optionally some DNA from immediately downstream of the 35K signal sequence, a multiple cloning site for introducing the foreign gene sequence and optionally the viral transcription terminator, in that order. Referring to Figure 2, by way of example, the plasmid "p1" (arbitrary designation) contains non-essential region left-hand sequence, promoter, signal sequence, a multiple cloning site having restriction sites W, X, Y and Z (four are shown, but the term "multiple" means two or more) and non-essential region right-hand sequence. (The above-recited optional additional elements have been omitted for clarity of illustration). X is a restriction site which enables the sequence X-X from plasmid "p2" to be inserted in the cloning site, resulting in the recombination plasmid "p3".

Using such a plasmid construct, it can be expected that recombination would take place in the non-essential region (NER) of the poxvirus genome. Preferably, therefore, the NER is of the same poxvirus, and, if possible, the same strain as is intended for the expression of the foreign gene. Moreover, some NERs might be substantially homologous in several poxviruses, in which case the NER need not be of the homologous virus. Precise homology of the NER of the recombination vector with that of the infecting poxvirus, i.e. that in which recombination is to take place, is not necessarily required. Examples of suitable NERs are for vaccinia virus the TK gene or 35K gene (this being non-essential and within the terminal inverted repeat region) and for fowlpox virus (FPV) a non-essential region within the inverted terminal repeat (ITR) as described in NRDC's UK Patent Application Publication No. 2220941A (or the equivalent PCT Application Publication No. WO85/12684, but the genomic location of the NER is not critical. Where it is within a ITR two copies of the foreign gene enter the poxvirus genome.

Theoretically, at least, homologous recombination does not require a NER, but can take place within an essential gene so long as the promoter signal sequence and foreign DNA are inserted into a non-essential region.

Hence, the NER can itself be flanked by a region which is in an essential gene. Preferably the cassette contains at least 1000 bp of flanking sequence, i.e. the NER sequence and optional further flanking sequence, at each end thereof.

Accordingly, the preferred construct (recombination vector insert) conveniently comprises:

(1) a first homologously recombinable sequence of a poxvirus genome;

(2) a sequence within the first portion of a non-essential region (NER) of the poxvirus genome;

(3) promoter DNA;

(4) signal sequence DNA of the invention transcribably linked to the promoter, i.e. linked in such a way that transcription can occur;

(5) foreign DNA in frame with the signal sequence DNA;

(preferably also 5A; a transcription termination signal compatible with the poxvirus);

(6) a sequence within a second portion of the said NER; and

(7) a second homologously recombinable sequence of the poxvirus genome.

Such a construct is cloned into a suitable vector such as a bacterial plasmid vector, to produce the required recombination vector.

Sequences (1) and (2) are not necessarily distinct, nor are (6) and (7). The termination signal might be important for the production of mature mRNA.

Recombination to produce the recombinant VV or other poxvirus can take place by any method known in VV recombinant technology and essentially comprises introducing an appropriate strain of poxvirus into animal cells in vitro and also introducing the recombination vector of the invention into those cells. Any animal cells infectable by VV will suffice, e.g. rat, rabbit, chicken or even human.

The principal use contemplated for the invention is in the in vitro synthesis of proteins, using vaccinia virus or another virus as a vector for the introduction into animal cells of foreign DNA to be expressed as protein. It is, however, also possible that incorporation of a signal sequence might help in the in vivo presentation of the foreign product in an animal which is to be vaccinated with a recombinant poxvirus, e.g. poultry with fowlpox virus.

The foreign protein thus produced will probably require enzymatic treatment to remove any amino acids of the mature 35kD VV protein fused to the foreign protein sequence, and/or to remove glycosylation. Any conventional such treatment can be used. The invention includes the foreign protein whether directly or indirectly obtained from the animal cells.

The following Example illustrates the invention.

## Example 1

Approach to mapping the gene encoding the 35kD polypeptide

The strategy used to map the gene encoding the 35kD polypeptide involved several stages:

(i) Initially the 35kD polypeptide was purified and used to develop an antiserum which was capable of recognizing the product of the 35K gene amongst the polypeptides synthesized from infected cell mRNA.

(ii) Specific DNA fragments from throughout the vaccinia virus genome were tested for their ability to arrest the in vitro translation of the 35kD protein.

(iii) A small (2.2 kb) DNA fragment which arrested translation was identified and its DNA sequence determined. This allowed the location of the 35K gene to be determined precisely, and the sequence of the encoded polypeptide to be deduced.

Production of antisera

The Lister strain of vaccinia virus is widely available.

Rabbit kidney (RK) cells were infected with the virus and 2.5 h after virus addition washed extensively with serum-free phosphate buffered saline (PBS). The proteins secreted into PBS during the next 3-4 h were collected and subjected to DEAE cellulose ion exchange chromatography on a column of Whatman DE52. A preparation of the 35kD protein was obtained which contained only small amounts of contaminating proteins.

The DEAE cellulose column-purified preparation was used to immunise rabbits, and an antiserum obtained which was able to precipitate the 35kD protein secreted.

Hybrid arrested translation of infected cell mRNA

mRNA from cells infected with strain Lister virus was translated in vitro using a rabbit reticulocyte lysate and the polypeptides were precipitated with the antiserum. Two in vitro products were efficiently immunoprecipitated. Using the Cleveland digest method, D.W. Cleveland et al ., J. Biol. Chem. 252 , 1102-1106 (1977), it was possible to identify one of these (designated IPa) as the in vitro precursor of the mature, secreted 35kD protein.

In vitro translations and immunoprecipitations were then performed using mRNA which had been hybridized to DNA fragments representing various regions of the vaccinia virus genome. In initial experiments, using the procedure of B.M. Paterson et al ., Proc. Natl. Acad. Sci. USA 74 4370-4374 (1977) the terminal Hind III fragments B and G (Figure 1) arrested translation of the 35K precursor. Testing of cloned subfragments allowed the identification of a 2.2 kb Bam HI plus Sal I fragment which also efficiently arrested translation and therefore contained the sequences of the 35K gene. Since this fragment lies entirely within the inverted terminal repeats there are in fact two copies of the 35K gene per viral genome.

Figure 1 shows the location of the 35K gene on the vaccinia virus genome. The boxed regions indicate the inverted terminal repeat sequences and the positions of the terminal Hind III (H) fragments B and G are shown. The expanded regions show the two copies of the 2.2 kb Bam HI (Ba) plus Sal I (S) fragment which arrested translation of the 35K gene (arrowed). 'Ss' indicates Sst I sites.

Nucleotide sequence of the 35K gene

The DNA sequence of the entire 2.2 kb Bam HI plus Sal I fragment plus approximately 300bp downstream of the Sal I site was determined by using the dideoxy chain termination method and is shown below, (SEQUENCE ID NO: 4).

GGATCCGACC CTAATTGCGC CGACGAGGAT GAACTCACTT CTCTTCATTA CTACTGTAAA 60
BamHI

CACATATCCA CGTTCTACGA AAGCAATTAT TACAAGTCAA GTCACACTAA GATGCGAGCC 120

GAGAAGCGAT TCATCTACGC GATAATAGAT CATGGAGCAA ACATTAACGC GGTTACACAC 180

TTACCTTCAA CAGTATACCA AACATAGTCC TCGTGTGGTG TATGCTCTTT TATCTCGAGG 240

ATACGTAATA ATCTTGATTG TACACCATCA TGGAACGATT GTGCAACAGG TCATATTCTC 300

ATAATGTTAC TCAATTGGCA CGAACAAAAG GAAGAAGGAC AACATCTACT TTATCTATTC 360

ATAAAACATA ATCAAGGATA CACTCTCAAT ATACTACGGT ATCTACTAGA TAGGTTCGAC 420

ATTCAGAAAG ACGAATACTA TAATACCGCC TTTCAAAATT GTAACAACAA TGTTGCCTCA 480

TACATCGGAT ACGACATCAA CCTTCCGACT AAAGACGGTA TTCGACTTGG TGTTTGAAAA 540

CAGAAACATC ATATACAAGG CGGATGTTGT GAATGACATC ATCCACCACA GACTGAAAGT 600

ATCTCTACCT ATGATTAAAT CGTTGTTCTA CAAGATGTCT CTCCCTACGA CGATTACTAC 660

GTAAAAAAGA TACTAGCCTA CTGCCTATTA AGGGACGAGT CATTCGCGGA ACTACATAGT 720

AAATTCTGTT TAAACGAGGA CTATAAAAGT GTATTTATGA AAAATATATC ATTCGATAAG 780

ATAGATTCCA TCATCGTGAC ATAAGTCGCC TTAAAGAGAT TCGAATCTCC GACACCGACC 840

TGTATACGGT ATCACAGCTA TCTTAAAGCC ATACATTCAG ACAGACACAT TTCATTTCCC 900

ATGTACGACG ATCTCAAACC CGTACCCAGA AATACCTTTA ACTATATCGA TGTGGAAATT 960
ClaI

AATCTGTATC CCGTCAACGA CACATCGTGT ACTCGGACGA CCACTACCGG TCTCAGCGAA 1020

TCCATCTCAA CGTCGGAACT AACTATTACT ATGAATCATA AAGACTGTAA TCCCGTCTTT 1080

CGTGATGGAT ACTTCTC[illegible]T CCTTAATAAG GTAGCAACTT CAGGTTTCTT TACAGGAGAA 1140

AGGTGTGCAC TCTGAATTTC GAGATTAAAT GCAATAACAA AGATTCTTCC TCCAAACAGT 1200

TAACGAAAGC AAAGAATGAT ACTATCATGC CGCATTCGGA GACAGTAACT CTAGTGGGCG 1260

ACATCTATAT ACTATATAGT AATACCAATA CTCAAGACTA CGAAACTGAT ACAATCTCTT 1320

ATCATGTGGG TAATGTTCTC GATGTCGATA GCCATATGCC CGGTAGTTGC GATATACATA 1380

AACTGATCAC TAATTCCAAA CCCACCCACT TTTTATAGTA AGTTTTTCAC CCATAAATAA 1440

```
                                                                 |→ mRNA
                                                                 |
TAAATACAAT AATTAATTTC TCGTAAAAGT AGAAAATATA TTCTAATTTA TTGCACGGTA   1500

5' end (approx).
AGGAAGTAGA ATCATAAAGA ACAGTACTCA ATCAATAGCA ATT ATG AAA CAA TAT     1555
                                                Met Lys Gln Tyr
                                                        -15
                                                     *
ATC GTC CTG GCA TGC ATG TGC CTG GCG GCA GCT GCT ATG CCT GCC AGT     1603
Ile Val Leu Ala Cys Met Cys Leu Ala Ala Ala Ala Met Pro Ala Ser
            -10                 -5                   1

CTT CAG CAA TCA TCC TCA TCC TCC TCC TCG TGT ACG GAA GAA GAA AAC     1651
Leu Gln Gln Ser Ser Ser Ser Ser Ser Ser Cys Thr Glu Glu Glu Asn
    5                   10                  15
                        ClaI
AAA CAT CAT ATG GGA ATC GAT GTT ATT ATC AAA GTC ACA AAG CAA GAC     1699
Lys His His Met Gly Ile Asp Val Ile Ile Lys Val Thr Lys Gln Asp
20                  25                  30                  35

CAA ACA CCG ACC AAT GAT AAG ATT TGC CAA TCC GTA ACG GAA ATT ACA     1747
Gln Thr Pro Thr Asn Asp Lys Ile Cys Gln Ser Val Thr Glu Ile Thr
                40                  45                  50

GAG TCC GAG TCA GAT CCA GAT CCC GAG GTG GAA TCA GAA GAT GAT TCC     1795
Glu Ser Glu Ser Asp Pro Asp Pro Glu Val Glu Ser Glu Asp Asp Ser
            55                  60                  65

ACA TCA GTC GAG GAT GTA GAT CCT CCT ACC ACT TAT TAC TCC ATC ATC     1843
Thr Ser Val Glu Asp Val Asp Pro Pro Thr Thr Tyr Tyr Ser Ile Ile
        70                  75                  80

GGT GGA GGT CTG AGA ATG AAC TTT GGA TTC ACC AAA TGT CCT CAG ATT     1891
Gly Gly Gly Leu Arg Met Asn Phe Gly Phe Thr Lys Cys Pro Gln Ile
    85                  90                  95

AAA TCC ATC TCA GAA TCC GCT GAT GGA AAC ACA GTG AAT GCT AGA TTG     1939
Lys Ser Ile Ser Glu Ser Ala Asp Gly Asn Thr Val Asn Ala Arg Leu
100                 105                 110                 115

TCC AGC GTG TCC CCA GGA CAA GGT AAG GAC TCT CCC GCG ATC ACT CGT     1987
Ser Ser Val Ser Pro Gly Gln Gly Lys Asp Ser Pro Ala Ile Thr Arg
                120                 125                 130

GAA GAA GCT CTT GCT ATG ATC AAA GAC TGT GAG GTG TCT ATC GAC ATC     2035
Glu Glu Ala Leu Ala Met Ile Lys Asp Cys Glu Val Ser Ile Asp Ile
            135                 140                 145

AGA TGT AGC GAA GAA GAG AAA GAC AGC GAC ATC AAG ACC CAT CCA GTA     2083
Arg Cys Ser Glu Glu Glu Lys Asp Ser Asp Ile Lys Thr His Pro Val
        150                 155                 160
```

```
CTC GGG TCT AAC ATC TCT CAT AAG AAA GTG AGT TAC GAA GAT ATC ATC    2131
Leu Gly Ser Asn Ile Ser His Lys Lys Val Ser Tyr Glu Asp Ile Ile
    165                 170                 175

GGT TCA ACG ATC GTC GAT ACA AAA TGT GTC AAG AAT CTA GAG TTT AGC    2179
Gly Ser Thr Ile Val Asp Thr Lys Cys Val Lys Asn Leu Glu Phe Ser
180                 185                 190                 195

GTT CGT ATC GGA GAC ATG TGC AAG GAA TCA TCT GAA CTT GAG GTC AAG    2227
Val Arg Ile Gly Asp Met Cys Lys Glu Ser Ser Glu Leu Glu Val Lys
                200                 205                 210
                          SalI
GAT GGA TTC AAG TAT GTC GAC GGA TCG GCA TCT GAA GGT GCA ACC GAT    2275
Asp Gly Phe Lys Tyr Val Asp Gly Ser Ala Ser Glu Gly Ala Thr Asp
            215                 220                 225

                    ClaI
GAT ACT TCA CTC ATC GAT TCA ACA AAA CTC AAA GCG TGT GTC TGA        2320
Asp Thr Ser Leu Ile Asp Ser Thr Lys Leu Lys Ala Cys Val ---
        230                 235                 240
ATCGATAACT CTATTCATCT GAAATTGGAT GAGTAGGGTT AATCGAACGA TTCAGGCACA  2380
ClaI

CCACGAATTA AAAAAGTGTA CCGGACACTA TATTCCGGTT TGCAAAACAA AAATGTTCTT  2440

AACTACATTC ACAAAAAGTT ACCTCTCGCG ACTTCTTCTT TTTCTGTCTC AATAGTGTGA  2500

TACGATTATG ACACTATTCC TATTCCTATT CCTATTTCCT TTCAGGGTAT CACAAAAATA  2560
                    mRNA   3'end

TTAAACCTCT TTCTGAT                                                 2577
```

**Note**: The published sequence for strain WR corresponds to residues 1267-1605 and 2405-2570. The counterpart in the above sequence of the 137 bp upstream of the start codon identified by Cochran *et al.*, *loc. cit.* as containing the VV 7.5K promoter in WR strain would be from 1407 to 1543 (immediately preceding the start codon at 1544).

The longest open reading frame was 235 amino acids and extended beyond the Sal I site at its C terminus. The N-terminal end of the predicted polypeptide encodes a hydrophobic region of 17 amino acids which functions as a signal sequence. Immediately beyond this the predicted amino acid sequence is (SEQUENCE ID NO: 5):

Pro Ala Ser Leu Gln Gln.

This sequence corresponds to the limited amino acid sequence for the N-terminus of DE52 column-purified mature 35kD protein (Unknown-Ala-Unknown-Leu-Gln-Gln) which had previously been obtained for us, in confidence, by J.E. Fothergill and B. Dunbar of the University of Aberdeen, making us confident that the sequenced gene encodes the secreted 35kD protein. The remainder of the 35K gene (downstream of the Sal I site) was determined by sequencing an overlapping cloned DNA fragment and the complete gene encodes a further 23 amino acids.

The sequence of the 2.2 kb Bam HI plus Sal I fragment is shown together with approximately 300 residues downstream of the Sal I site. The amino acids encoded by the 35K open reading frame are indicated. The 19 residues missing from strain WR DNA are underlined (1580-1598 inclusive) and the asterisked proline indicates the position (amino acid number 1) of the N-terminus of the mature secreted 35kD protein (i.e. following cleavage of the signal sequence). Also shown are the mapped positions of the 5′ and 3′ ends of the strain WR 7.5K mRNA (Venkatesan et al ., loc . cit .) The underlined restriction enzyme recognition sites are those for Cla I, Bam HI and Sal I (see Example 2).

## Example 2

This Example relates to the testing of the promoter and signal sequences.

### Construction of recombination vectors carrying cassette DNA from the 35K gene region and foreign genes for insertion into the 35K gene.

Figure 3 shows a linearised map of the plasmids used in the construction of cassette DNA comprising promoter, signal sequence (omitted in a comparative construct) and foreign gene. Referring to Figure 3, the Bam HI-Sal I sub-fragment of a terminal Hind III fragment was cloned in the widely available plasmid pUC19. The resulting plasmid, which has most of the coding sequence for the 35kD protein, was designated pG62. The remainder of the 35K gene is located on an overlapping Xba I fragment in plasmid p48-15. PG620 was constructed from these two plasmids and carries the Bam HI to Xba I fragment which includes the entire 35K gene.

The Cla I fragments coding for all except the first 42 N-terminal amino acids of the 35kD precursor polypeptide were deleted as follows. pG62 was cleaved with Hind III and partially with Cla I. A DNA fragment carrying the pUC19 and the vaccinia DNA sequence starting from the Bam HI to the second Cla I site was isolated from an agarose gel by electroelution and ligated to the Cla I and Hind III fragment of the plasmid p48-15 forming pD35.

A Bgl II linker was introduced in the Cla I site downstream from the 35K promoter in pD35 forming pD35-L. The vaccinia DNA sequence in pD35-L was then subcloned on an Eco RI to Sal I fragment into pAT153 cleaved with the same enzymes. The resulting plasmid PD351 carries the DNA sequence left of the 35K gene [35K(L)], the 35K promoter (P35K) and the coding sequence for 42 N-terminal amino acids (42 aa of the precursor polypeptide, including 17 aa signal sequence) followed by a unique Bgl II site and the DNA sequence right of the 35K gene [35K(R)]. The lac Z gene and the cat genes, each on a Bam HI fragment, were cloned separately into the Bgl II site of pD351 to form plasmids pD352 and pD351/CAT respectively. These constructs have the above marker genes linked in frame to DNA encoding the N-terminal 42 amino acids of the 35K precursor polypeptide and contain the 35K promoter.

For comparative purposes, a vector for introducing the lac Z and the cat gene immediately downstream from the ATG translation initiation codon of the 35K gene, was constructed. Such a vector lacks the signal sequence but contains the promoter linked to the foreign gene. pD351 was cleaved with Bgl II, the ends filled in and partially digested with Sca I. The DNA fragment carrying the 35K(R) sequence starting from Bgl II/filled in end, PAT153 and the 35K(L) sequence ending at Sca I site 18bp upstream from the ATG start codon was isolated from an agarose gel by electroelution and ligated to a linker (SEQUENCE ID NO: 6):-

ACTCAATCAA TAGCAATTAT GGATCC

to form pD356. This allowed us to generate a Bam HI site immediately downstream from the ATG codon and also to delete the N-terminal coding sequence of the 35K gene in pD351. The sequence upstream from the ATG codon remained unchanged. In the newly created Bam HI site, the Bam HI DNA fragments encoding the lac Z and cat genes were inserted separately to form pD357 and pD356/CAT respectively. These constructs have the first ATG codon of the 35K gene linked in frame to the marker genes.

In order to construct a vector which allowed in frame translational fusions of the two marker genes to only the DNA encoding the first 22 N-terminal amino acids (which contain the putative signal peptide) of the 35K precursor polypeptide, the Eco RI and Bam HI fragment of pD356 carrying 35K(R) sequence and pAT 153 was ligated to the Eco RI and Sph I fragment of pG62 carrying the 35K(L), the 35K promoter plus part of the coding sequence (first 7 amino acids) of the precursor polypeptide in the presence of an oligonucleotide linker with Sph I and Bam HI sticky ends carrying the coding sequence downstream from the Sph I site (amino acids -10-5, followed by the Bam HI site) to form pD358. The lac Z and cat fragments were inserted separately into the newly formed Bam HI site in pD358, forming plasmids pD359 and pD358/CAT respectively.

Construction of recombination vectors for insertion of cassette DNA into vaccinia virus in the TK gene

Referring now to the linearised plasmid map of Figure 4, recombination vectors were constructed which allowed insertion of the marker genes, linked to the 35K promoter, into the TK (thymidine kinase non-essential gene) locus of the VV genome. A Bam HI site was introduced by synthesising a linker of formula:

AATTGGATCC
CCTAGGTTAA

and inserting this linker into the Eco RI site located within the TK gene in plasmid pV32 to form pV321 (pV32 is pAT153 carrying the Hind III K fragment of the Lister strain DNA; the corresponding fragment of the WR strain genome contains the TK gene). An approximately 270 bp Bcl I and Bgl II fragment of pD351 specifying the 35K promoter and the coding sequence for the N-terminal 42 amino acids (42 aa) of the precursor polypeptide was inserted together with the Bam HI cat fragment into the newly generated Bam HI site in the TK locus of pV321. This new recombination vector pV325 gave in frame linkage which would enable fusion of the cat gene to the N-terminal 42 amino acids expressed from the 35K gene, under the control of the 35K promoter, for insertion into the TK locus of the vaccinia genome. TK(L) and TK(R) refer to segments of vaccinia DNA which include the left and right portion of the TK gene.

The above Bcl I and Bam HI fragment of pD351 was also inserted into the Bam HI site between the 11K vaccinia promoter (P11K) and the lac Z gene of pSC8 [pSC8 has the lac Z gene under the control of the 11K VV late promoter flanked on both sides by vaccinia TK sequences; S. Chakrabarti et al . Mol. and Cell. Biol. S, 3403-3409 (1985)], to construct pSC8/351. This plasmid has the lac Z gene fused to DNA encoding the N-terminal 42 amino acids of the 35K precursor polypeptide and under the control of the 35K promoter. The 11K promoter is upstream from the 35K promoter.

An approximately 170 bp Bcl I and Bam HI fragment of the comparative cassette vector pD356 carrying the 35K promoter sequences and the ATG start codon was cloned into the Bam HI site in the TK locus in pV321 to form pV326. The Bam HI DNA fragments encoding the lac Z and cat genes were inserted separately into the Bam HI site immediately downstream of the ATG initiation codon in pV326 to form pV327 and pV328 respectively. These plasmids contain the 35K promoter but lack the signal sequence and were prepared for comparative purposes.

An approximately 230 bp Bc II plus Bam HI fragment of the comparative cassette vector pD358 carrying the 35K promoter and sequences encoding the first 22 amino acids from the N-terminus of the precursor polypeptide was cloned into the Bam HI site in the TK locus in pV321 to form pV329. The Bam HI cat fragment was then inserted in frame into the Bam HI site downstream of the sequences encoding the 35K promoter and 22 amino acid signal sequence to form pV331.

Construction and analysis of recombinant vaccinia viruses carrying the lacZ gene

Calcium phosphate-precipitated plasmids pD357 and pV327 (containing the lac Z gene linked to the 35K promoter but no signal sequence and inserted into the 35K and TK loci, respectively) were transfected into CV-1 cells infected with wild type vaccinia virus strain Lister. Recombinant viruses (blue plaques) were isolated as reported previously [Mackett et al ., J. Virol. 49 , 857-864 (1984); Chakrabarti et al ., Mol. Cell. Biol. 5 , 3403-3409 (1985)].

Recombinant viruses containing the lac Z gene under the control of the 35K promoter either in the 35K locus or TK gene (V357 and V327, respectively) efficiently expressed beta-galactosidase activity. As expected, most of the activity was located intracellularly. Southern blot analysis confirmed that the lac Z gene had been inserted into the 35K locus in both inverted terminal repeats of the V357 genome.

Cells infected with V357 did not express detectable 35kD protein in either the medium or cell extract. In order to exclude the possibility that expression of lac Z might inhibit synthesis of the 35kD protein in trans , it was shown that the recombinant virus V327 carrying the same 35K promoter-lac Z fusion inserted into the TK locus retained the ability to express and secrete the 35kD protein. These results indicate that the failure of V357 to express the 35kD protein is due to deletion of the 35K gene. They confirm that the 35kD secreted protein is encoded by the open reading frame identified in Example 1, and show that its presence is not essential for the growth of vaccinia virus strain Lister in tissue culture.

Construction of recombinant viruses carrying the cat gene

Plasmids pV328, pV331 pV325 and pD351-CAT were used to construct the recombinant viruses V328, V331, V325 and V351-CAT respectively, as described above. The gene was inserted into the TK locus of strain Lister in viruses V328, V331 and V325 and into both copies of the 35K gene in V351-CAT. V328, V331, V325 and V351-CAT respectively contain sequences encoding 0, 22, 42 and 42 amino acids of the precursor polypeptide linked in frame to the 5′ end of the cat gene.

Secretion of CAT by recombinant viruses

Secreted (SP) and intracellular (CE) CAT protein and CAT enzyme activity were determined 6h post-infection of CV1 cells with the above recombinant viruses. The results are shown in table 1:

TABLE 1

| Secretion of CAT polypeptide and enzyme activity by recombinant viruses | | | | |
|---|---|---|---|---|
| VIRUS | ELISA assay ng CAT protein/$2x10^5$ cells | | CAT activity nmole of chloramphenicol acetylated/$2x10^5$ cells | |
| | SP | CE | SP | CE |
| V328 | 0.138 | 65.50 | 1.335 | 205.150 |
| V331 | 2.000 | 2.60 | 8.825 | 1.420 |
| V325 | 2.930 | 1.85 | 0.975 | 0.343 |
| V351-CAT | 3.400 | 1.00 | 4.095 | 1.350 |
| Note: The ELISA and enzyme activity data are taken from separate experiments. Measurements were performed on extracellular medium (SP) or intracellular extracts (CE) following harvesting at 6 h post-infection. | | | | |

Table 1 shows that although much more CAT protein was produced by the control virus, V328, than by the other viruses, considerably more was secreted from cells infected with V331, V325 and V351-CAT (see results of ELISA assay). Similarly measurement of CAT enzyme activity in a separate experiment showed that the majority of the detectable activity was secreted from cells infected with V331, V325 and V351-CAT, but remained intracellular in V328 infected cells. These results indicate that fragments containing 22 and 42 amino acids from the N-terminus of the 35kD precursor polypeptide include functional signal sequences and are able to direct efficient secretion of the normally largely intracellular CAT protein.

Effect of tunicamycin on secretion of CAT

A reproducible observation in experiments of the type described above was that the CAT protein produced by recombinant viruses V331, V325 and V351-CAT exhibited a lower specific activity (i.e. per ng CAT protein) than that produced by V328. It was considered that this might possibly be due to the former proteins becoming glycosylated as a result of the signal sequence directing the CAT protein through the secretory pathway where glycosylation is known to occur. Moreover, it was noted that the CAT protein contains a potential site for N-linked glycosylation Asn-Gln-Thr at positions 34-36. To test this hypothesis, tunicamycin, which inhibits N-linked glycosylation of proteins, was used. CAT activity was measured in the medium (SP) and intracellular extracts (CE) of CV-1 cells infected for 18 h with the CAT recombinant viruses in the presence or absence of tunicamycin.

The results are shown in table 2:

TABLE 2

| Effect of tunicamycin on the secretion of CAT | | | | |
|---|---|---|---|---|
| VIRUS | CAT activity nmoles of chloramphenicol acetylated/2x$10^5$ cells | | | |
| | SP | | CE | |
| | +T | -T | +T | -T |
| V328 | 1.16 | 3.01 | 2328.30 | 2573.60 |
| V331 | 155.30 | 51.78 | 171.80 | 16.00 |
| V325 | 338.40 | 12.50 | 199.20 | 7.40 |
| Note: Cells were infected in the presence (+T) or absence (-T) of tunicamycin, and CAT activities determined for the medium (SP) and intracellular extracts (CE). | | | | |

The results (table 2) show that tunicamycin increased the level of CAT activity produced by V331 and V325 but not by V328. Protein gels indicated that tunicamycin caused a small decrease in the amount of CAT protein synthesised. Therefore the specific activity of the fusion proteins specified by V331 and V325 is substantially increased in the presence of tunicamycin. This is consistent with the reduced activities of the CAT protein specified by these viruses being at least partly due to inhibition by glycosylation. The increase in intracellular activity detected in the presence of tunicamycin in cells infected with V331 and V325 suggests that the intracellular enzyme activity is in transit through the secretory pathway.

Oligonucleotide-directed mutagenesis of the cat gene

The cat gene was specifically mutated so as to remove the one potential N-linked glycosylation site Asn-Gln-Thr at amino acid positions 34-36. The amino acids Asn-34 and Thr-36 were substituted with Gln and Val respectively as follows. The Bam HI fragment carrying the cat gene was subcloned into vector pTZ19U (Pharmacia), and a single stranded DNA template form of this plasmid, pTZ19U-CAT, was propagated in E . coli strain TG1 with the phage M13K07 as helper [Mead et al ., Protein Engineering 1 , 67-76 (1986)]. An oligonucleotide (SEQUENCE ID NO: 7):

3′ CGTGTTACAT GGATA G T T GT C CA GCAAGTC GACCTATAA 5′

carrying mismatched bases (underlined) was synthesised. Mutagenesis of the cat DNA was performed according to the phosphorothioate based method of Sayers and Eckstein (1989, In Protein Function: A Practical Approach p279-295. Edited by Creighton, T.E. IRL Press, Oxford). A plasmid (pTZ19U-CAT14) containing substitution mutations coverting Asn-34 to Gln and Thr-36 to Val was identified by DNA sequencing. The mutant cat gene on the Bam HI fragment of pTZ19U-CAT14 was subsequently cloned in the correct orientation into recombination vectors pV326 and pV329 to form plasmids pV328-1 and pV331-10 respectively. An approximately 270 bp Bcl I and Bgl II fragment of pD351 specifying the 35K promoter and the coding sequences for the N-terminal 42 amino acids was inserted together with the Bam HI mutant cat fragment from pTZ19U-CAT14 into the TK locus of pV321 to form plasmid pV325-11. The plasmids pV328-1, pV331-10 and pV325-11 are identical to pV328, pV331 and pV325 respectively except for the two amino acids substitutions disrupting the potential N-linked glycosylation site. The mutant cat constructs were introduced into the TK locus of the strain Lister genome by homologous recombination, and the recombinant viruses V328-1, V331-10 and V325-11 were isolated as described above.

Analysis of the CAT activity produced by viruses carrying mutated cat genes

BHK cells were infected in the presence or absence of tunicamycin with the recombinant viruses carrying the mutated and wild type cat gene constructs, and secreted and intracellular CAT activities analysed after 18 h incubation. The results are shown in table 3:

TABLE 3

| Analysis of viruses carrying mutated cat genes | | | | |
|---|---|---|---|---|
| VIRUS | CAT activity nmoles of chloraphenicol acetylated/6x10$^5$ cells | | | |
| | -Tunicamycin | | +Tunicamycin | |
| | SP | CE | SP | CE |
| V328 | 78.300 | 10214.20 | 45.020 | 4323.700 |
| V328-1 | 0.145 | 160.00 | 0.037 | 55.840 |
| V331 | 57.700 | 57.30 | 230.490 | 713.900 |
| V331-10 | 0.038 | 0.56 | 0.017 | 0.358 |
| V325 | 35.130 | 40.10 | 650.630 | 838.440 |
| V325-11 | 0.058 | 2.13 | 0.019 | 1.038 |
| Note: Cells were infected in the presence or absence of tunicamycin and CAT activities determined in the extracellular medium (SP) and intracellular extracts (CE). | | | | |

Table 3 shows that the level of CAT activity produced by V328-1, V331-10 and V325-11 is not elevated by tunicamycin. On the contrary, in the presence of tunicamycin there appeared to be some decrease in CAT activity encoded by these viruses, possibly due to a toxic effect of the drug on cells. In the absence of the drug, the mutated CAT proteins expressed by viruses V331-10 and V325-11 migrated faster during polyacrylamide gel electrophoresis than their non-mutated counterparts (produced by V331 and V325). This is consistent with the specific mutations eliminating a glycosylation site. Strikingly, these amino acid substitutions also caused a severe reduction of overall CAT activities encoded by V328-1, V331-10 and V325-11 (table 3). Other results show that the quantity of the mutated CAT proteins expressed by viruses V328-1, V331-10 and V325-11 are similar to that expressed by their non-mutated counterparts. The results therefore strongly suggest that it is possible to abolish glycosylation of secreted CAT by specific mutations, although in this case these mutations unfortunately seem to reduce the enzymatic activity of the CAT protein (possibly due to an effect on the active site).

In summary, the N-terminal 22 and 42 amino acids of the precursor polypeptide are both able to function as signal sequences and efficiently direct secretion of proteins to which they are attached. However, because they route the hybrid proteins through the secretory pathway, one potential problem is that proteins that are not normally glycosylated may become so if they possess glycosylation sites. The results show that there is a possibility of circumventing this problem either by using tunicamycin or by removing the glycosylation sites by site-specific mutagenesis of the gene encoding the protein to be secreted.

This sequence listing is provided in this International Patent Application to meet the requirements or wishes of certain contracting States (EPC countries, US, JP). The "General Information Section" is applicable only to US.

SEQUENCE LISTING

(1) GENERAL INFORMATION

(i) Applicant : GAFFNEY, Dareina Frances; PATEL, Arvind Hirabhai; STOW, Nigel Dennis; SUBAK-SHAREP, John Herbert

(ii) Title of invention : DNA coding for a polypeptide signal sequence in vaccinia virus.

(iii) Number of Sequences : 7

(iv) Correspondence Address : Nixon & Vanderhye,
14th Floor,
2200 Clarendon Boulevard,
Arlington,
Virginia, U.S.A. 22201

(v) Computer Readable Form :

(A) MEDIUM : Diskette, 5.25 inch, 360 Kb storage
(B) COMPUTER : IBM PC/AT compatible
(C) OPERATING SYSTEM : MS-DOS 3.2
(D) SOFTWARE : WordPerfect ASCII File Format

(vi) Current Application Data :

(A) APPLICATION NUMBER :
(B) FILING DATE :
(C) CLASSIFICATION :

(vii) Prior Application Data :

(A) APPLICATION NUMBER : PCT/GB 90/
(B) FILING DATE :

(A) APPLICATION NUMBER : GB 8915807.3
(B) FILING DATE : 11th July 1989

(viii) Agent/Attorney Information : Leonard C. Mitchard
Reg'n No. 29009
Reference Docket No.

(ix) Telecommunication Information :

(A) TELEPHONE : (703) 875-0400
(B) TELEFAX : (703) 525 3468

(2) INFORMATION FOR SEQUENCE ID NO: 1

(i) Sequence Characteristics :

| | | |
|---|---|---|
| (A) Length | : | 60 base pairs corresponding to 20 amino acids |
| (B) Type | : | Nucleotide with corresponding amino acids |
| (C) Strandedness | : | Double |
| (D) Topology | : | Linear |

| | | |
|---|---|---|
| (ii) Original Source | : | Vaccinia Virus, Lister strain |
| (iii) Experimental Source | : | Vaccinia Virus, Lister strain |
| (iv) Properties | : | DNA encoding a signal sequence which enables a polypeptide to pass through cell membranes, plus the N-terminal sequence of the secreted polypeptide. |

(v) SEQUENCE ID NO: 1:

```
ATG AAA CAA TAT ATC GTC CTG GCA TGC ATG    30
Met Lys Gln Tyr Ile Val Leu Ala Cys Met
        -15                 -10

TGC CTG GCG GCA GCT GCT ATG CCT GCC AGT    60
Cys Leu Ala Ala Ala Ala Met Pro Ala Ser
        -5                   1
```

(3) INFORMATION FOR SEQUENCE ID NO: 2

(i) Sequence Characteristics :

| | | |
|---|---|---|
| (A) Length | : | 20 amino acids |
| (B) Type | : | Amino acid sequence |
| (D) Topology | : | Linear |

| | | |
|---|---|---|
| (ii) Original Source | : | Vaccinia virus, Lister strain |
| (iii) Experimental Source | : | Vaccinia virus, Lister strain |
| (iv) Properties | : | Signal sequence enabling a polypeptide to pass through cell membranes, plus the N-terminal amino acids of the secreted polypeptide. |

(v) SEQUENCE ID NO: 2 :

```
Met Lys Gln Tyr Ile Val Leu Ala Cys Met
        -15                 -10

Cys Leu Ala Ala Ala Ala Met Pro Ala Ser
        -5                   1
```

(4) INFORMATION FOR SEQUENCE ID NO: 3

(i) Sequence Characteristics:

(A) Sequence Length : 14 Amino acids
(B) Sequence Type : Amino acid sequence
(D) Topology : Linear

(ii) Original Source : 35K gene of Vaccinia Virus, Lister strain

(iii) Experimental Source: 35K gene of Vaccinia Virus, Lister strain

(iv) Fragments: Amino acid residues 4-17 of the N terminus of the 35kD protein of Vaccinia Virus, Lister strain

(iv) SEQUENCE ID NO: 3

```
Leu Gln Gln Ser Ser Ser Ser Ser Ser Ser
     5                  10

Cys Thr Glu Glu
    15
```

(5) INFORMATION FOR SEQUENCE ID NO: 4

(i) Sequence characteristics

| | | |
|---|---|---|
| (A) Sequence Length | : | 2577 base pairs |
| (B) Sequence Type | : | Nucleotide sequence and corresponding protein sequence |
| (C) Strandedness | : | Double |
| (D) Topology | : | Linear |
| (E) Molecule Type | : | Genomic DNA |

(ii) Original Source : Vaccinia Virus, Lister strain

(iii) Experimental source : Vaccinia Virus, Lister strain

(iv) Properties : Fragment of Inverted Terminal Repeat of Vaccinia Virus DNA, Lister strain. Contains the 35K gene (2 copies per genome).

(v) Features : From 1544 to 2317 encodes the 35K polypeptide.

(vi) SEQUENCE ID NO: 4 :

```
GGATCCGACC CTAATTGCGC CGACGAGGAT GAACTCACTT CTCTTCATTA CTACTGTAAA    60
CACATATCCA CGTTCTACGA AAGCAATTAT TACAAGTCAA GTCACACTAA GATGCGAGCC   120
GAGAAGCGAT TCATCTACGC GATAATAGAT CATGGAGCAA ACATTAACGC GGTTACACAC   180
TTACCTTCAA CAGTATACCA AACATAGTCC TCGTGTGGTG TATGCTCTTT TATCTCGAGG   240
ATACGTAATA ATCTTGATTG TACACCATCA TGGAACGATT GTGCAACAGG TCATATTCTC   300
ATAATGTTAC TCAATTGGCA CGAACAAAAG GAAGAAGGAC AACATCTACT TTATCTATTC   360
ATAAAACATA ATCAAGGATA CACTCTCAAT ATACTACGGT ATCTACTAGA TAGGTTCGAC   420
ATTCAGAAAG ACGAATACTA TAATACCGCC TTTCAAAATT GTAACAACAA TGTTGCCTCA   480
TACATCGGAT ACGACATCAA CCTTCCGACT AAAGACGGTA TTCGACTTGG TGTTTGAAAA   540
CAGAAACATC ATATACAAGG CGGATGTTGT GAATGACATC ATCCACCACA GACTGAAAGT   600
ATCTCTACCT ATGATTAAAT CGTTGTTCTA CAAGATGTCT CTCCCTACGA CGATTACTAC   660
GTAAAAAAGA TACTAGCCTA CTGCCTATTA AGGGACGAGT CATTCGCGGA ACTACATAGT   720
AAATTCTGTT TAAACGAGGA CTATAAAAGT GTATTTATGA AAAATATATC ATTCGATAAG   780
ATAGATTCCA TCATCGTGAC ATAAGTCGCC TTAAAGAGAT TCGAATCTCC GACACCGACC   840
TGTATACGGT ATCACAGCTA TCTTAAAGCC ATACATTCAG ACAGACACAT TTCATTTCCC   900
ATGTACGACG ATCTCAAACC CGTACCCAGA AATACCTTTA ACTATATCGA TGTGGAAATT   960
AATCTGTATC CCGTCAACGA CACATCGTGT ACTCGGACGA CCACTACCGG TCTCAGCGAA  1020
TCCATCTCAA CGTCGGAACT AACTATTACT ATGAATCATA AAGACTGTAA TCCCGTCTTT  1080
CGTGATGGAT ACTTCTCTGT CCTTAATAAG GTAGCAACTT CAGGTTTCTT TACAGGAGAA  1140
AGGTGTGCAC TCTGAATTTC GAGATTAAAT GCAATAACAA AGATTCTTCC TCCAAACAGT  1200
TAACGAAAGC AAAGAATGAT ACTATCATGC CGCATTCGGA GACAGTAACT CTAGTGGGCG  1260
ACATCTATAT ACTATATAGT AATACCAATA CTCAAGACTA CGAAACTGAT ACAATCTCTT  1320
ATCATGTGGG TAATGTTCTC GATGTCGATA GCCATATGCC CGGTAGTTGC GATATACATA  1380
AACTGATCAC TAATTCCAAA CCCACCCACT TTTTATAGTA AGTTTTTCAC CCATAAATAA  1440
TAAATACAAT AATTAATTTC TCGTAAAAGT AGAAAATATA TTCTAATTTA TTGCACGGTA  1500
AGGAAGTAGA ATCATAAAGA ACAGTACTCA ATCAATAGCA ATT ATG AAA CAA TAT    1555
                                                Met Lys Gln Tyr
                                                        -15
```

*

ATC GTC CTG GCA TGC ATG TGC CTG GCG GCA GCT GCT ATG CCT GCC AGT 1603
Ile Val Leu Ala Cys Met Cys Leu Ala Ala Ala Ala Met Pro Ala Ser
-10 -5 1

CTT CAG CAA TCA TCC TCA TCC TCC TCC TCG TGT ACG GAA GAA GAA AAC 1651
Leu Gln Gln Ser Ser Ser Ser Ser Ser Ser Cys Thr Glu Glu Glu Asn
5 10 15

AAA CAT CAT ATG GGA ATC GAT GTT ATT ATC AAA GTC ACA AAG CAA GAC 1699
Lys His His Met Gly Ile Asp Val Ile Ile Lys Val Thr Lys Gln Asp
20 25 30 35

CAA ACA CCG ACC AAT GAT AAG ATT TGC CAA TCC GTA ACG GAA ATT ACA 1747
Gln Thr Pro Thr Asn Asp Lys Ile Cys Gln Ser Val Thr Glu Ile Thr
40 45 50

GAG TCC GAG TCA GAT CCA GAT CCC GAG GTG GAA TCA GAA GAT GAT TCC 1795
Glu Ser Glu Ser Asp Pro Asp Pro Glu Val Glu Ser Glu Asp Asp Ser
55 60 65

ACA TCA GTC GAG GAT GTA GAT CCT CCT ACC ACT TAT TAC TCC ATC ATC 1843
Thr Ser Val Glu Asp Val Asp Pro Pro Thr Thr Tyr Tyr Ser Ile Ile
70 75 80

GGT GGA GGT CTG AGA ATG AAC TTT GGA TTC ACC AAA TGT CCT CAG ATT 1891
Gly Gly Gly Leu Arg Met Asn Phe Gly Phe Thr Lys Cys Pro Gln Ile
85 90 95

AAA TCC ATC TCA GAA TCC GCT GAT GGA AAC ACA GTG AAT GCT AGA TTG 1939
Lys Ser Ile Ser Glu Ser Ala Asp Gly Asn Thr Val Asn Ala Arg Leu
100 105 110 115

TCC AGC GTG TCC CCA GGA CAA GGT AAG GAC TCT CCC GCG ATC ACT CGT 1987
Ser Ser Val Ser Pro Gly Gln Gly Lys Asp Ser Pro Ala Ile Thr Arg
120 125 130

GAA GAA GCT CTT GCT ATG ATC AAA GAC TGT GAG GTG TCT ATC GAC ATC 2035
Glu Glu Ala Leu Ala Met Ile Lys Asp Cys Glu Val Ser Ile Asp Ile
135 140 145

AGA TGT AGC GAA GAA GAG AAA GAC AGC GAC ATC AAG ACC CAT CCA GTA 2083
Arg Cys Ser Glu Glu Glu Lys Asp Ser Asp Ile Lys Thr His Pro Val
150 155 160

CTC GGG TCT AAC ATC TCT CAT AAG AAA GTG AGT TAC GAA GAT ATC ATC 2131
Leu Gly Ser Asn Ile Ser His Lys Lys Val Ser Tyr Glu Asp Ile Ile
165 170 175

GGT TCA ACG ATC GTC GAT ACA AAA TGT GTC AAG AAT CTA GAG TTT AGC 2179
Gly Ser Thr Ile Val Asp Thr Lys Cys Val Lys Asn Leu Glu Phe Ser
180 185 190 195

```
GTT CGT ATC GGA GAC ATG TGC AAG GAA TCA TCT GAA CTT GAG GTC AAG    2227
Val Arg Ile Gly Asp Met Cys Lys Glu Ser Ser Glu Leu Glu Val Lys
                200                 205                 210

GAT GGA TTC AAG TAT GTC GAC GGA TCG GCA TCT GAA GGT GCA ACC GAT    2275
Asp Gly Phe Lys Tyr Val Asp Gly Ser Ala Ser Glu Gly Ala Thr Asp
            215                 220                 225

GAT ACT TCA CTC ATC GAT TCA ACA AAA CTC AAA GCG TGT GTC TGA        2320
Asp Thr Ser Leu Ile Asp Ser Thr Lys Leu Lys Ala Cys Val ---
        230                 235                 240

ATCGATAACT CTATTCATCT GAAATTGGAT GAGTAGGGTT AATCGAACGA TTCAGGCACA  2380

CCACGAATTA AAAAAGTGTA CCGGACACTA TATTCCGGTT TGCAAAACAA AAATGTTCTT  2440

AACTACATTC ACAAAAAGTT ACCTCTCGCG ACTTCTTCTT TTTCTGTCTC AATAGTGTGA  2500

TACGATTATG ACACTATTCC TATTCCTATT CCTATTTCCT TTCAGGGTAT CACAAAAATA  2560

TTAAACCTCT TTCTGAT                                                 2577
```

(6) INFORMATION FOR SEQUENCE ID NO: 5

(i) Sequence characteristics:

(A) Sequence Length : 6 amino acids

(B) Sequence Type : Amino acid sequence

(D) Topology : Linear

(ii) Original Source : 35K polypeptide of Vaccinia Virus, Lister strain.

(iii) Experimental Source : 35K polypeptide of Vaccinia Virus, Lister strain.

(iv) Fragments : Amino acid residues 1-6 of the N-terminus of the 35kD protein of Vaccinia Virus, Lister strain.

(v) SEQUENCE ID NO. 5 :

```
Pro Ala Ser Leu Gln Gln
1                   6
```

(7) INFORMATION FOR SEQUENCE ID NO: 6

(i) Sequence characteriscs:

| | | |
|---|---|---|
| (A) Sequence Length | : | 26 base pairs |
| (B) Sequence Type | : | Nucleotide sequence |
| (C) Strandedness | : | Double |
| (D) Topology | : | Linear |

(ii) Properties : Oligonucleotide

(iii) SEQUENCE ID NO. 6

ACTCAATCAA TAGCAATTAT GGATCC 26

(8) INFORMATION FOR SEQUENCE ID NO: 7

(i) Sequence characteristics:

| | | |
|---|---|---|
| (A) Sequence Length | : | 39 bases |
| (B) Sequence Type | : | Nucleic acid |
| (C) Strandedness | : | Single |
| (D) Topology | : | Linear |

(ii) Properties : Oligonucleotide

(iii) SEQUENCE ID NO: 7:

AATATCCAGC TGAACGACCT GTTGATAGGT ACATTGTGC 39

## Claims

1. Signal sequence DNA which encodes the following amino acid sequence (SEQUENCE ID NO: 2):

| | | | | | | | | | |
|---|---|---|---|---|---|---|---|---|---|
| Met | Lys | Gln | Tyr | Ile | Val | Leu | Ala | Cys | Met |
| Cys | Leu | Ala | Ala | Ala | Ala | Met | Pro | Ala | Ser |

or a conservatively modified variant of said amino acid sequence.

2. DNA according to claim 1 wherein the amino acid sequence further comprises (following on from the C-terminal end of SEQUENCE ID NO: 1): Leu Gln, optionally another Gln and further optionally Ser.

3. A DNA cassette suitable for insertion into the genome of a poxvirus, said cassette comprising (in order from 5′ to 3′ end) a promoter sequence transcribably linked to a signal sequence defined in claim 1 or 2, followed by a multiple cloning site for in-frame insertion of a foreign gene.

4. A DNA cassette according to claim 3 which further comprises a foreign gene downstream of the multiple cloning site, in frame with the signal sequence.

5. A recombination vector carrying cassette DNA according to claim 4, with flanking poxvirus non-essential region sequence at each end thereof, for homologous recombination into a poxvirus genome.

6. A recombination vector according to claim 5 which comprises:

(1) a first homologously recombinable sequence of a poxvirus genome;

(2) a sequence within the first portion of a non-essential region (NER) of the poxvirus genome;

(3) promoter DNA;

(4) signal sequence DNA as defined in claim 1 or 2 transcribably linked to the promoter;

(5) foreign DNA in frame with the signal sequence DNA;

(6) a sequence within a second portion of the said NER; and

(7) a second homologously recombinable sequence of the poxvirus genome.

7. A recombination vector according to Claim 6 which includes a transcription termination signal compatible with the poxvirus between the foreign DNA (5) and the second NER region (6).

8. Animal cells infected with a poxvirus and a recombination vector according to claim 5, 6 or 7.

9. A foreign protein produced from animal cells claimed in Claim 8.

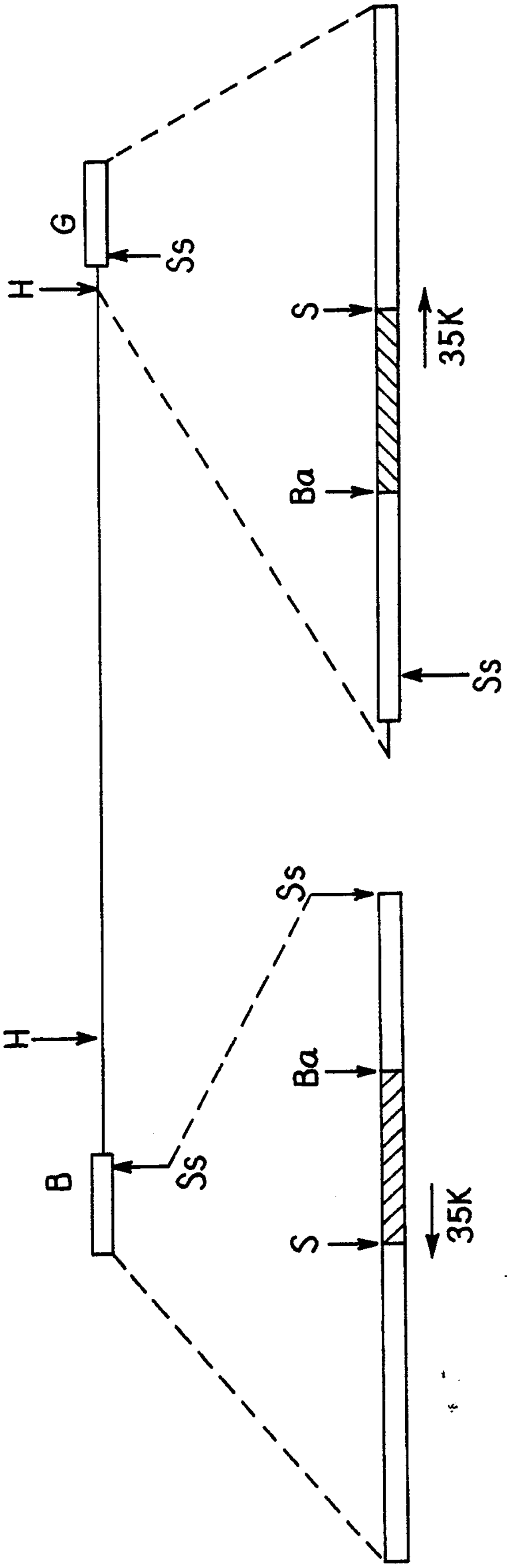


Fig.1

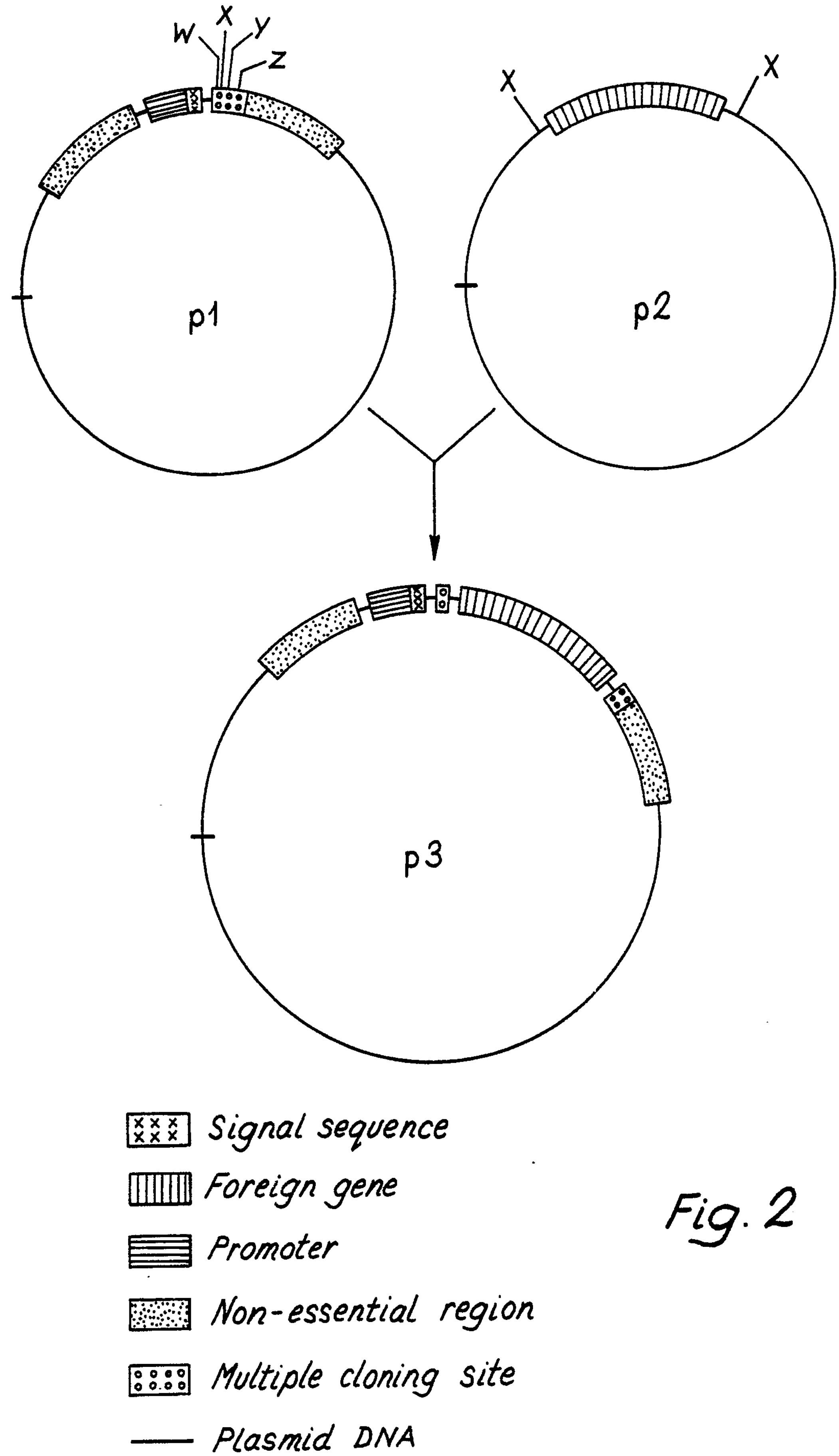
W
X
Y
Z
X
X
p1
p2
p3
Signal sequence
Foreign gene
Promoter
Non-essential region
Multiple cloning site
Plasmid DNA

Fig. 2

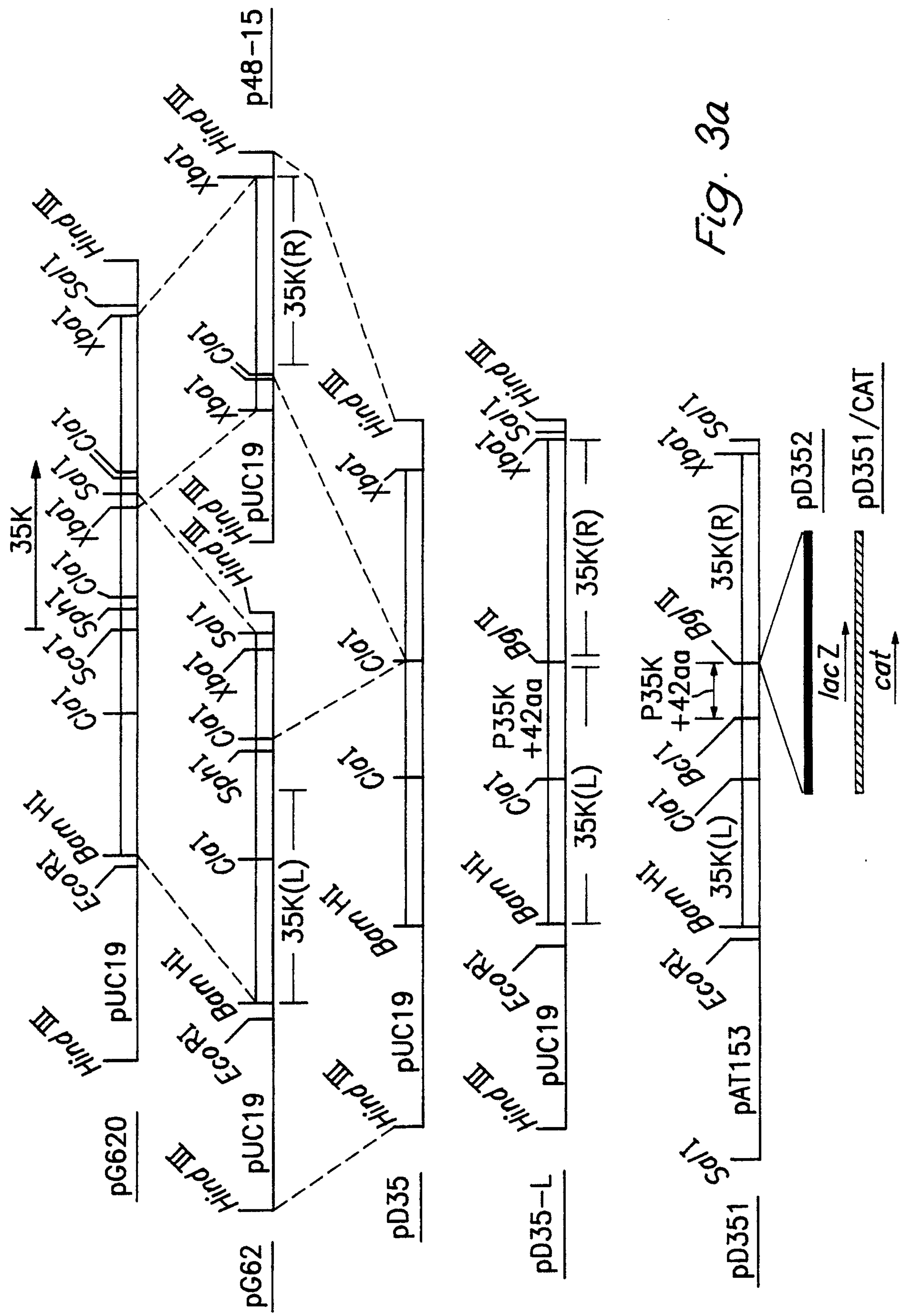
pG620
pG62
pD35
pD35-L
pD351
p48-15
pD352
pD351/CAT
pUC19
pAT153
35K
35K(L)
35K(R)
P35K
+42aa
HindIII
EcoRI
BamHI
ClaI
ScaI
SphI
XbaI
SalI
BglII
BclI
lacZ
cat

Fig. 3a

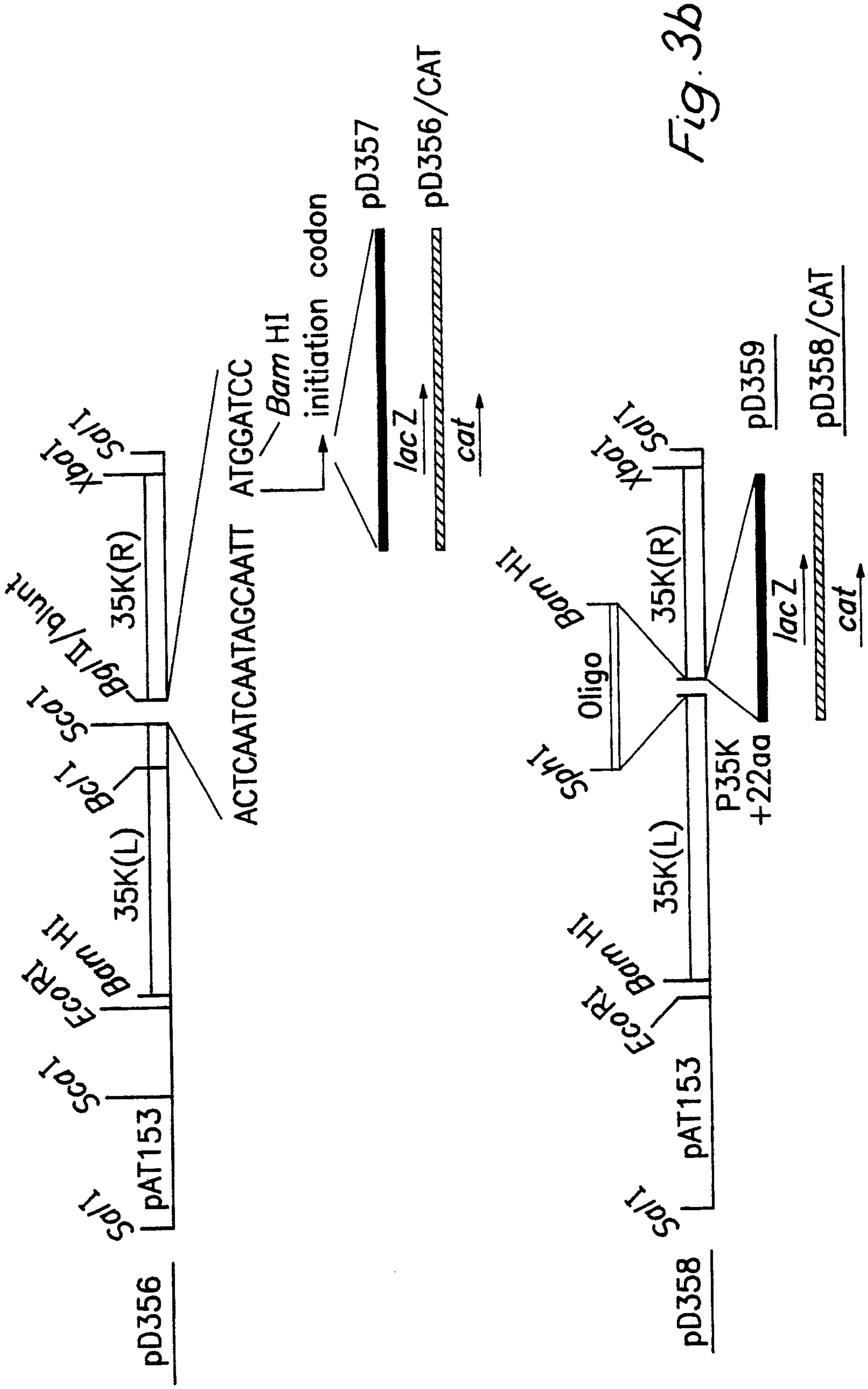
pD356
SalI
pAT153
ScaI
EcoRI
Bam HI
35K(L)
BclI
ScaI
BglII/blunt
35K(R)
XbaI
SalI
ACTCAATCAATAGCAATT ATGGATCC
Bam HI
initiation codon
pD357
lacZ
pD356/CAT
cat
pD358
SalI
pAT153
EcoRI
Bam HI
35K(L)
SphI
Oligo
Bam HI
P35K
+22aa
35K(R)
XbaI
SalI
lacZ
cat
pD359
pD358/CAT

Fig. 3b

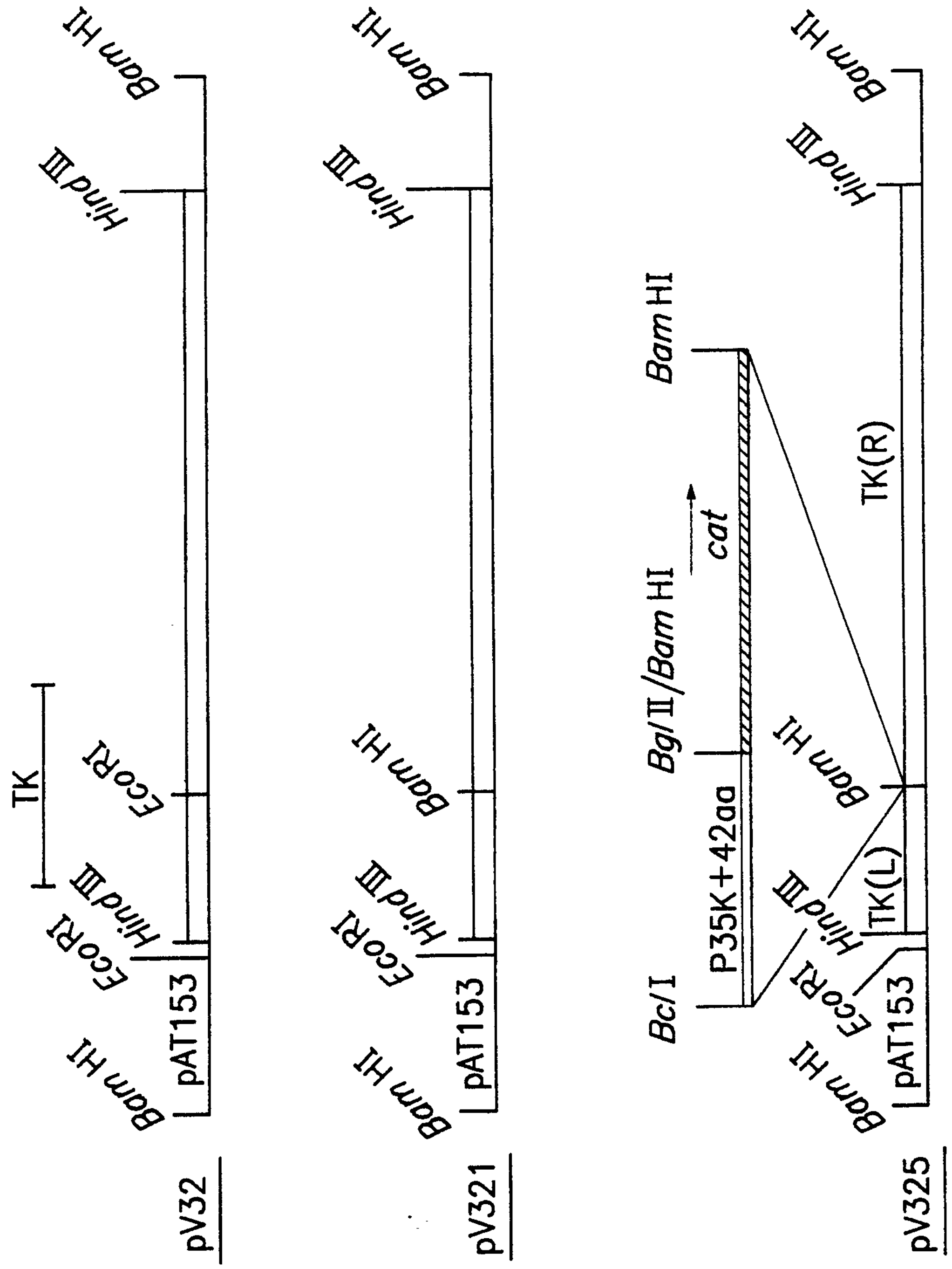

pV32
pV321
pV325
pAT153
TK
TK(L)
TK(R)
Bam HI
EcoRI
Hind III
BclI
BglII/Bam HI
P35K+42aa
cat


Fig. 4a

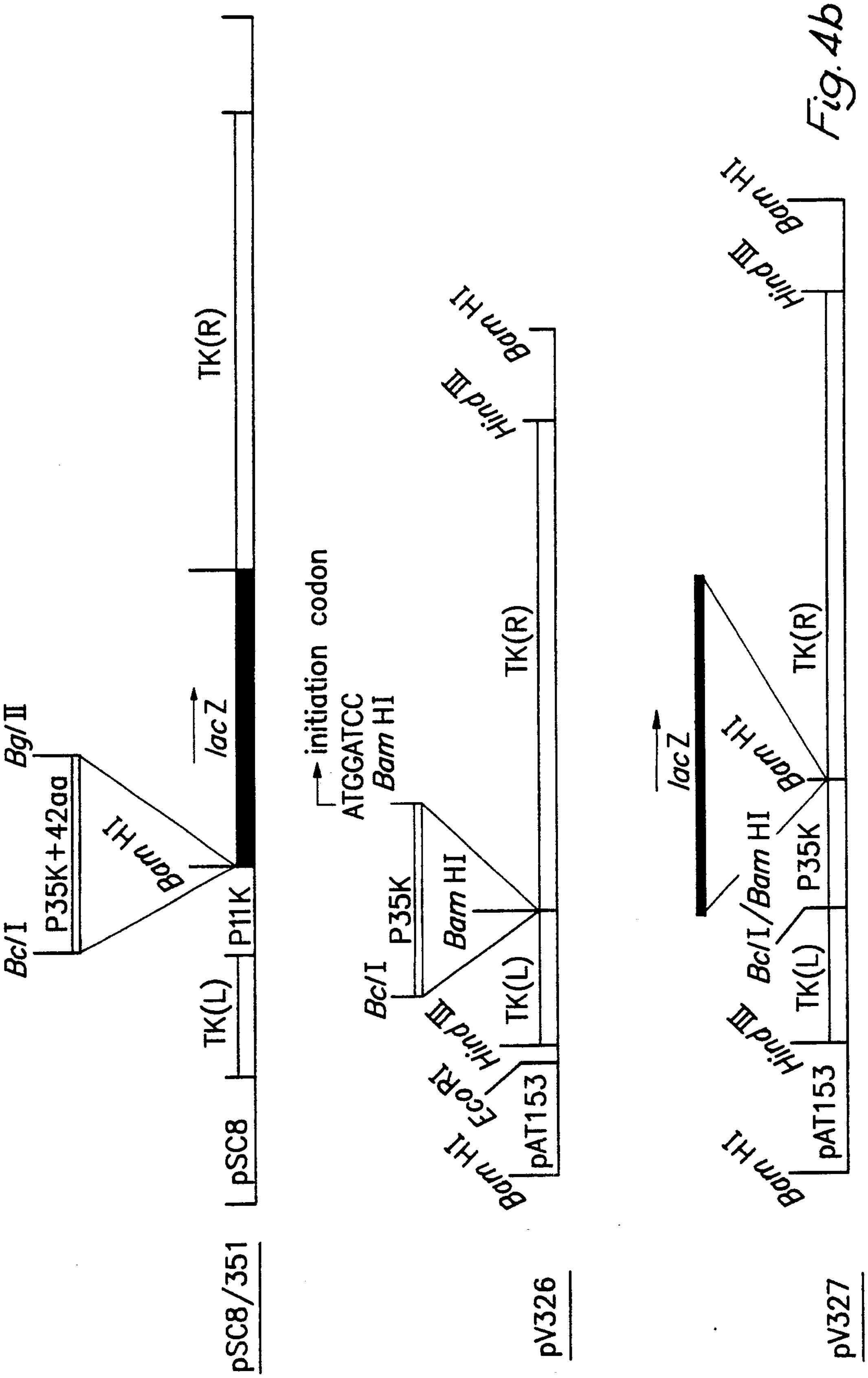
pSC8/351
pSC8
TK(L)
P11K
BclI
P35K+42aa
BglII
Bam HI
lacZ
TK(R)
pV326
Bam HI
pAT153
EcoRI
Hind III
TK(L)
BclI
P35K
Bam HI
ATGGATCC
initiation codon
TK(R)
pV327
lacZ
BclI/Bam HI


Fig. 4b(1)

Fig. 4b (2)

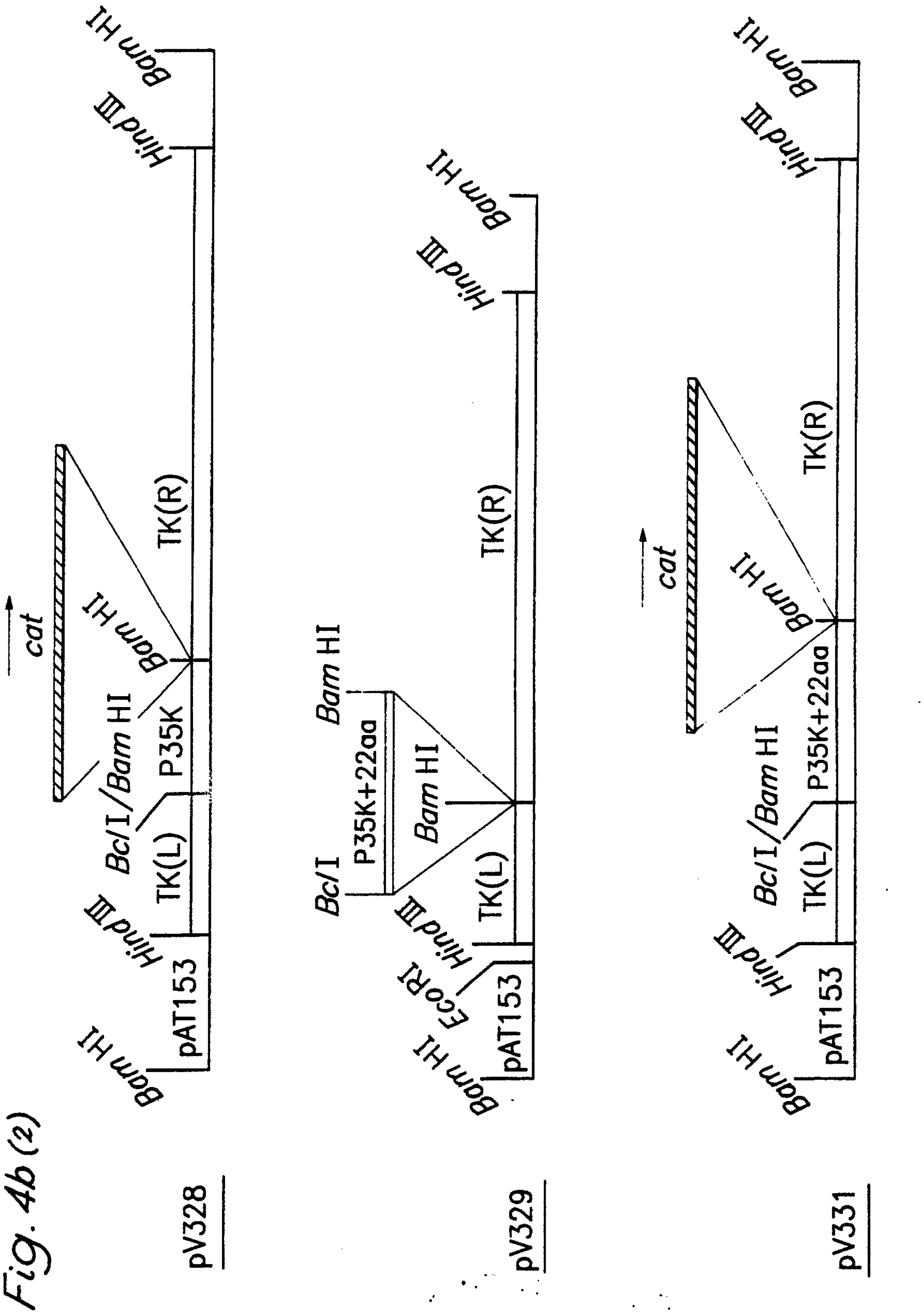
pV328
Bam HI
pAT153
Hind III
TK(L)
Bcl I/Bam HI
P35K
Bam HI
cat
TK(R)
Hind III
Bam HI
pV329
Bam HI
pAT153
EcoRI
Hind III
TK(L)
Bcl I
P35K+22aa
Bam HI
Bam HI
TK(R)
Hind III
Bam HI
pV331
Bam HI
pAT153
Hind III
TK(L)
Bcl I/Bam HI
P35K+22aa
Bam HI
cat
TK(R)
Hind III
Bam HI

European Patent Office

# EUROPEAN SEARCH REPORT

Application Number

EP 90 30 7513

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | WO-A-8607609 (TRANSGENE S.A.)<br>* page 3, lines 4 - 15 *<br>--- | 1-9 | C12N15/62<br>C12N15/86<br>C12N15/39 |
| A | JOURNAL OF GENERAL VIROLOGY<br>vol. 67, no. 10, 1986,<br>pages 2067 - 2082; MACKETT, M. & SMITH, G.L.:<br>"Vaccinia virus expression vectors."<br>* the whole document *<br>----- | 1-9 | |
| | | | TECHNICAL FIELDS SEARCHED (Int. Cl.5 ) |
| | | | C12N |

The present search report has been drawn up for all claims

| Place of search | Date of completion of the search | Examiner |
|---|---|---|
| THE HAGUE | 07 SEPTEMBER 1990 | ANDRES S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document